(19) Europäisches Patentamt
European Patent Office
Office européen des brevets

(11) Veröffentlichungsnummer: **0 032 540**
**B1**

(12) # EUROPÄISCHE PATENTSCHRIFT

(45) Veröffentlichungstag der Patentschrift: **23.11.83**

(51) Int. Cl.³: **C 07 D 295/12** // A61K49/04

(21) Anmeldenummer: **80107244.8**

(22) Anmeldetag: **20.11.80**

(54) **2-Methyl-3-(2,4,6-trijod-3-(1-morpholinoäthylidenamino)-benzamido)-propionitril, Verfahren zu dessen Herstellung und dessen Verwendung als Zwischenprodukt.**

(30) Priorität: **04.01.80 DE 3000209**

(43) Veröffentlichungstag der Anmeldung: **29.07.81 Patentblatt 81/30**

(45) Bekanntmachung des Hinweises auf die Patenterteilung: **23.11.83 Patentblatt 83/47**

(84) Benannte Vertragsstaaten: **AT BE CH DE FR GB IT LI LU NL SE**

(56) Entgegenhaltungen:
**AT - B - 292 724**
**DE - C - 2 235 935**

(73) Patentinhaber: **CHEMIE LINZ AKTIENGESELLSCHAFT, St. Peter-Strasse 25, A-4020 Linz (AT)**
(84) Benannte Vertragsstaaten: **BE CH FR GB IT LI LU NL SE AT**

(73) Patentinhaber: **Lentia Gesellschaft mit beschränkter Haftung, Arabellastrasse 4 Postfach 81 05 08, D-8000 München 81 (DE)**
(84) Benannte Vertragsstaaten: **DE**

(72) Erfinder: **Wieser, Josef, Dr., Boschweg 1c, A-4020 Linz (AT)**
Erfinder: **Krieger, Josef, Denkstrasse 25, A-4020 Linz (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# 2-Methyl-3-[2,4,6-trijod-3-(1-morpholinoäthylidenamino)-benzamido]-propionitril, Verfahren zu dessen Herstellung und dessen Verwendung als Zwischenprodukt

Die vorliegende Erfindung betrifft das neue 2-Methyl-3-[2,4,6-trijod-3-(1-morpholinoäthylidenamino)-benzamido]-propionitril, ein Verfahren zu dessen Herstellung und dessen Verwendung zur Herstellung der 2-Methyl-3-[2,4,6-trijod-3-(1-morpholinoäthylidenamino)-benzamido]-propionsäure, die als Wirkstoff in Mitteln für die perorale Schnellcholecystographie bekannt ist.

Aus der DE-PS 2 235 935 ist bekannt, dass Derivate von 2,4,6-Trijod-benzamido-alkancarbonsäuren, die in 3-Stellung des Benzolkerns eine substituierte Amidinogruppe tragen, Röntgenkontrastmittel zur Sichtbarmachung der Gallenblase sind, die sich besonders durch ihre gute Resorbierbarkeit und rasche Eliminierung aus dem Körper auszeichnen. Unter diesen Verbindungen hat besonders 2-Methyl-3-[2,4,6-trijod-3-(1-morpholinoäthylidenamino)-benzamido]-propionsäure (Iomorinsäure) der Formel I

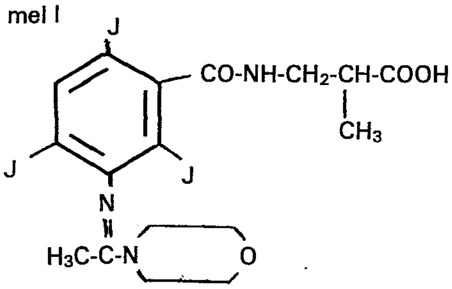

(I)

Bedeutung erlangt, die als sogenanntes Schnellcholecystographiemittel eingesetzt wird. Nach peroraler Gabe dieser Verbindung, bevorzugt in Form des Na-Salzes, können nach 60 bis 90 Minuten Bilder der Gallengänge und binnen 5 Stunden solche der Gallenblase erhalten werden, so dass Einnahme und Untersuchung an einem Tag möglich sind.

Zur Herstellung der Verbindung der Formel I wird gemäss DE-PS 2 235 935 2,4,6-Trijod-3-aminobenzoylchlorid mit N-Acethylmorpholin und Phosphoroxychlorid in Chloroform unter Kochen umgesetzt und das resultierende Säurechlorid mit gebildeter Amidinogruppe als Salz isoliert. Dieses kann dann nach Freisetzung der Base durch Umsetzung mit 2-Methyl-3-aminopropionsäureestern ebenfalls in der Hitze und anschliessende Verseifung in die Verbindung der Formel I übergeführt werden.

Dieses Verfahren hat den Nachteil, dass bei der Umsetzung des 2,4,6-Trijod-3-aminobenzoylchlorids mit dem Acetylmorpholin säureunlösliche Nebenprodukte und ausserdem als weiteres Nebenprodukt das Morpholid der 2,4,6-Trijod-3-(1-morpholinoäthylidenamino)-benzoesäure gebildet wird. Diese Verunreinigungen können erst nach Herstellung des Endprodukts abgetrennt werden, was sehr schwierig ist, weil die Säure der Formel I in verunreinigter Form schlecht oder gar nicht kristallisiert und sich die Reinigung sehr verlustreich gestaltet und ein mehrfaches Eindampfen von Mutterlaugen erforderlich macht.

Als weitere Verfahrensvariante wird in der genannten DE-PS auch die Umsetzung von 2,4,6-Trijod-3-aminobenzamido-alkancarbonsäureestern mit N-Acetylmorpholin und Phosphoroxychlorid, ebenfalls unter Kochen am Rückfluss, angegeben. Bei dieser Verfahrensweise kann zwar die Morpholidbildung vermieden werden, die Reaktion verläuft aber trotzdem nicht einheitlich und es entstehen auch hier säureunlösliche Nebenprodukte. Diese lassen sich zwar zum Grossteil durch Ansäuern auf pH 1-1,5 abtrennen, es bleiben aber doch gewisse Mengen dieser Nebenprodukte im Produkt und erschweren dessen Reinigung sehr.

Schliesslich ist es auch gemäss DE-PS 2 235 935 noch möglich, die Herstellung der Verbindung der Formel I bei Raumtemperatur durch Umsetzung von 2-Methyl-3-(2,4,6-trijod-3-acetylamino)-benzamidopropionsäuremethylester mit Morpholin in Gegenwart von Phosphorpentachlorid herzustellen, doch werden bei dieser Reaktionsweise noch mehr Nebenprodukte gebildet, so dass die Ausbeute an Reinprodukt noch niedriger und die Reinigung noch aufwendiger ist als bei den anderen bekannten Verfahren.

Es konnte nun im neuen Nitril der Verbindung der Formel I mit der Formel II

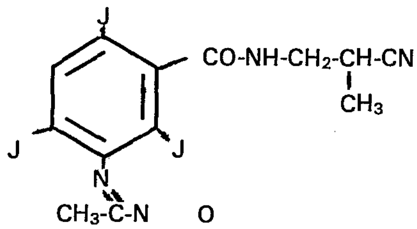

(II)

eine Verbindung gefunden werden, die eine gute Kristallisationstendenz zeigt und sich aufgrund ihrer guten Löslichkeit und Beständigkeit in verdünnten wässrigen Säuren und Schwerlöslichkeit der Base in Wasser sehr gut und auf einfache Weise reinigen lässt. Obwohl diese Verbindung also bedenkenlos zu Reinigungszwecken mehrmals umgefällt werden kann, lässt sie sich überraschenderweise sowohl mit starken Säuren als auch in alkalischer Lösung zur Säure der Formel I hydrolysieren, ohne dass die Amidinogruppe unter Bildung von schädlichen Verunreinigungen angegriffen oder eine Jodabspaltung erfolgen würde. Damit ist durch die Auffindung dieser neuen Substanz ein neuer, vorteilhafter Weg zur Herstellung der Verbindung der Formel I eröffnet worden, denn wenn man das reine Nitril der Formel II der Hydrolyse unterwirft, entsteht die Säure der Formel I in so reiner Form, dass maximal eine einfache Umkristallisation, z.B. aus Methanol, die ohne nennenswerte Verluste durchführbar ist, genügt, um die Säure in einer für die pharmazeutische Anwendung brauchbaren Reinheit zu erhalten. Da damit das verlustreiche Reinigen der Säure wegfällt und überdies sowohl die Herstellung des Nitrils der Formel II als auch dessen Verseifung in sehr guter Ausbeute gelingt, ist damit die Herstellung der Iomorinsäure der Formel I auch in wesentlich höherer Ausbeute möglich, als es bisher der Fall war.

Gegenstand der vorliegenden Erfindung ist demnach das neue Nitril der Iomorinsäure der Formel II, dessen Herstellung sowie dessen Verwendung zur Herstellung der Iomorinsäure der Formel I durch Hydrolyse.

Die Herstellung des Nitrils der Formel II erfolgt nach den für die Herstellung der Ester der Iomorinsäure an sich bekannten Methoden.

So kann man 2-Methyl-3-(2,4,6-trijod-3-aminobenzamido)-propionitril, vorzugsweise in einem inerten Reaktionsmedium, mit N-Acetylmorpholin in Gegenwart von Phosphoroxychlorid umsetzen. Durch Behandlung des resultierenden Reaktionsgemisches vor oder während des Abdampfens des gegebenenfalls verwendeten Lösungsmittels mit Wasser entsteht eine saure wässrige Lösung, die das Nitril der Formel II als Salz gelöst enthält. Eventuell vorhandene säureunlösliche Nebenprodukte können dann in fester Form z.B. durch Filtration auf einfache Weise abgetrennt werden. Neutralisiert man dann die klare saure Lösung und stellt einen pH-Wert von mindestens 8 ein, so fällt das schwerlösliche Nitril entweder gleich in kristallisierter Form oder aber als Öl aus, das sehr rasch durchkristallisiert und so leicht in fester Form gewonnen werden kann.

Es ist aber auch möglich, das Nitril der Formel II ausgehend von 2,4,6-Trijod-3-(1-morpholinoäthylidenamino)-benzoylchlorid durch Umsetzung mit 3-Aminoisobuttersäurenitril in einem inerten Lösungsmittel zu erhalten. Hier muss das Reaktionsprodukt nach Abdampfen des Lösungsmittels zuerst durch Behandlung mit wässriger Säure gelöst und anschliessend durch Alkalisieren ausgefällt werden.

Bei der Durchführung der Umsetzung von 2-Methyl-3-(2,4,6-trijod-3-aminobenzamido)-propionitril mit N-Acetylmorpholin und Phosphoroxychlorid, die die bevorzugte Verfahrensvariante darstellt, werden zweckmässig sowohl das N-Acetylmorpholin als auch das $POCl_3$ gegenüber dem Nitril im Überschuss angewendet. Zweckmässig setzt man 1,5-3 Mol N-Acetylmorpholin und 2-6 Mol Phosphoroxychlorid pro Mol des Nitrils ein. Dieser Überschuss an den beiden Reaktionspartnern unterdrückt die Bildung von Nebenprodukten und erhöht die Ausbeute, wobei man innerhalb der angegebenen Grenzen dann bessere Resultate erzielt, wenn man wenigstens einen der beiden Ausgangsstoffe in einer Menge entsprechend dem oberen Bereich der angegebenen Intervalle einsetzt. Unter 5% an Nebenprodukten und Ausbeuten an Nitril der Formel II von mehr als 95% werden erhalten, wenn man pro Mol 2-Methyl-3-(2,4,6-trijod-3-aminobenzamido)-propionitril 3 Mol N-Acetylmorpholin und 2-6 Mol $POCl_3$ einsetzt. Verwendet man hingegen nur 2 Mol N-Acetylmorpholin, so werden zur Erzielung optimaler Ergebnisse 6 Mol $POCl_3$ pro Mol 2-Methyl-3-(2,4,6-trijod-3-aminobenzamido)-propionitril benötigt. Die besten Ergebnisse werden bei einem Molverhältnis von 1:3:6 erhalten.

Die Reaktionstemperatur wird vorzugsweise möglichst tief, und zwar im Bereich zwischen 0°C und etwa 20°C, gehalten. Besonders günstig ist es, wenn beim Zusammenmischen der Reaktionspartner eine Temperatur von etwa 0°C eingestellt wird und die Temperatur erst zum Ausreagieren bis auf etwa 20°C ansteigen gelassen wird. Da die Reaktion exotherm ist, muss dazu gekühlt werden. Es ist aber auch möglich, bei höherer Temperatur, z.B. bei Siedetemperatur des Reaktionsgemisches, ausreagieren zu lassen, vor allem dann, wenn kürzere Reaktionszeiten erwünscht sind.

Die Tatsache, dass neben dem Nitril der Formel II noch Nebenprodukte anfallen, hat zwar Einfluss auf die Ausbeute, ist aber für das Gelingen des Verfahrens bzw. für die Reinheit des Endprodukts der Formel I ohne Bedeutung. Dies deshalb, weil sich das Nitril der Formel II aufgrund seiner Löslichkeitseigenschaften ausgezeichnet reinigen lässt. Das bedeutet, dass auch bei Durchführung des Verfahrens unter Bedingungen, bei denen mehr als 5% an Nebenprodukten erhalten werden, die Reinigung des Nitrils keine Schwierigkeiten bietet und daher die Reinheit des Endprodukts der Formel I trotzdem befriedigt.

Die Umsetzung des 2-Methyl-3-(2,4,6-trijod-3-aminobenzamido)-propionitrils mit dem N-Acetylmorpholin und $POCl_3$ wird vorzugsweise in einem inerten Reaktionsmedium, zweckmässig in einem indifferenten organischen Lösungsmittel, durchgeführt. Da das als Ausgangsmaterial dienende Nitril in den gebräuchlichen organischen Lösungsmitteln kaum löslich ist, wird meist in solchen Lösungsmitteln gearbeitet, in denen zwar der sich aus N-Acetylmorpholin und Phosphoroxychlorid bildende Komplex, nicht aber das Nitril löslich ist. Solche Lösungsmittel sind z.B. Dioxan, Tetrahydrofuran, Acetonitril und chlorierte Kohlenwasserstoffe wie Chloroform oder Methylenchlorid. Überraschenderweise ist trotz des Arbeitens in heterogener Phase die Ausbeute sehr gut.

Die Löslichkeit des Komplexes aus Acetylmorpholin und $POCl_3$ ist jedoch keine Voraussetzung für das Gelingen des erfindungsgemässen Verfahrens. Die Reaktion geht auch glatt in solchen organischen Lösungsmitteln, wie aromatische Kohlenwasserstoffe, insbesondere Toluol, vor sich, in denen der Komplex aus Acetylmorpholin und $POCl_3$ nicht löslich ist, sondern nur suspendiert wird. Die Reaktion kann auch in überschüssigem $POCl_3$ als Lösungsmittel durchgeführt werden. Zweckmässig ist es, die saure Lösung, die beim Aufnehmen des Reaktionsprodukts in Wasser entsteht, noch durch Zusatz von Tierkohle zu reinigen. Die Fällung der Base der Formel II geschieht dann durch Alkalisieren mit einer wasserlöslichen Base, die alkalisch genug ist, um das Erreichen eines pH-Wertes von mindestens 8 zu gewährleisten. Bevorzugt wird Ammoniak verwendet. Sind mehr Nebenprodukte vorhanden, kann ohne Schwierigkeit oder Verluste eine nochmalige Reinigung eingeschaltet werden, indem man das Nitril der Formel II nochmals in verdünnter Säure auflöst, evtl. noch einmal mit Aktivkohle behandelt und wieder durch Alkalisieren ausfällt.

Die zweite Verfahrensvariante, die Umsetzung des Säurechlorids mit bereits vorgebildeter Amidogruppe mit 3-Aminoisobuttersäurenitril, wird in einem inerten Lösungsmittel wie Dioxan, Chloroform oder Tetrahydrofuran, zweckmässig unter Erwärmen, durchgeführt. Auch hier lassen sich die säureunlöslichen Nebenprodukte durch die vorteilhaften Eigenschaften des Nitrils der Formel II unschwer abtrennen. Die Reinigung geschieht auch bei diesem Verfahren nötigenfalls durch mehrmaliges Lösen in Säure, Behandeln mit Aktivkohle und Fällen mit Alkalien, vorzugsweise Ammoniak.

Die Verseifung des auf diese Weise in reiner Form gewonnenen Nitrils der Formel II geht überraschenderweise völlig glatt vor sich. Sie kann durch Behandlung mit konzentrierten anorganischen Säuren wie Salzsäure oder Schwefelsäure durchgeführt werden. Sie ist aber auch mit Hilfe von alkalischen Mitteln, z.B. mit alkoholisch-wässrigen Alkalilaugen, wie NaOH oder KOH, möglich. Schliesslich kann aber auch zunächst durch Einwirkung von Säuren in alkoholischem Medium, z.B. mit methanolischer Salzsäure, der entsprechende Imidoester hergestellt und dieser durch Behandlung mit Wasser gespalten werden. Aus der Verseifungslösung wird die freie Verbindung der Formel I, die amphoteren Charakter besitzt, am besten am isoelektrischen Punkt gefällt, wobei sie in nahezu quantitativer Ausbeute in reiner Form erhalten wird.

Das als Ausgangsmaterial benötigte 2-Methyl-3-(2,4,6-trijod-3-aminobenzamido)-propionitril ist auf einfache Weise durch Acylierung von 3-Aminoisobuttersäurenitril durch 3-Amino-2,4,6-trijodbenzoylchlorid in Ausbeuten über 90% erhältlich. Das 3-Aminoisobuttersäurenitril wird durch Addition von Ammoniak an Methacrylnitril ebenfalls auf einfache Weise hergestellt und ist durch Destillation leicht rein zu erhalten.

*Beispiel 1*

183,6 g (1,2 Mol) Phosphoroxychlorid werden in 500 ml Chloroform bei einer Temperatur von 0°C mit 77,4 g (0,6 Mol) N-Acetylmorpholin versetzt. Dabei steigt die Temperatur trotz weiteren Kühlens auf etwa 8°C an. Nachdem die Temperatur wieder auf 0°C gefallen ist, werden 116,2 g (0,2 Mol) 2-Methyl-3-(2,4,6-trijod-3-aminobenzamido)-propionitril zugegeben, worauf ohne weitere Kühlung die Suspension gerührt wird.

Nach 40 Stunden wird die Chloroformsuspension in 800 ml Wasser eingegossen, und zwar in dem Masse, dass das Chloroform laufend abdestilliert und sich eine klare wässrige Phase bildet. Um die letzten Reste von Chloroform zu vertreiben, wird die wässrige Lösung noch kurz aufgekocht. Dann wird gekühlt, mit Kohle filtriert und die saure Lösung zuerst mit 40%iger wässriger Natronlauge auf pH 6 gebracht und dann mit konzentriertem wässrigem Ammoniak alkalisiert. Das zum Teil ölig ausfallende Nitril der Formel II wird bei Erwärmen der wässrig-ammoniakalischen Lösung auf 85°C und kräftigem Rühren nach kurzer Zeit fest und filtrierbar. Das filtrierte Produkt wird noch einmal in 1,2 l Wasser, welches 0,4 Mol Salzsäure enthält, gelöst, mit Kohle behandelt, mit Ammoniak wie oben beschrieben alkalisiert und ausgefällt.

Man erhält 137 g 2-Methyl-3-[2,4,6-trijod-3-(1-morpholinoäthylidenamino)-benzamido]-propionitril vom Fp: 129-130°C, das sind 98,9% der Theorie.

Das als Ausgangsmaterial benötigte 2-Methyl-3-(2,4,6-trijod-3-aminobenzamido)-propionitril wird wie folgt hergestellt: 106,6 g (0,2 Mol) 2,4,6-Trijod-3-aminobenzoesäurechlorid werden in 500 ml Chloroform mit 17,6 g 3-Aminoisobuttersäurenitril und 21,2 g Triäthylamin mehrere Stunden rückflussgekocht. Schon während des Erhitzens beginnt 2-Methyl-3-(2,4,6-trijod-3-aminobenzamido)-propionitril auszukristallisieren. Zur Vervollständigung der Kristallisation werden vom Reaktionsgemisch 200 ml Chloroform abdestilliert, dann wird das Gemisch bei Raumtemperatur stehengelassen. Nach Filtration erhält man 108,4 g (93,3%) des Nitrils. Aus der Mutterlauge werden nach Waschen des Chloroforms mit Wasser und anschliessendem Einengen auf 40 ml weitere 6,3 g (5,4%) erhalten.

Fp: 187°C.

*Beispiel 2*

183,6 g (1,2 Mol) Phosphoroxychlorid und 77,4 g (0,6 Mol) N-Acetylmorpholin werden bei 0°C in 500 ml Dioxan eingetragen und dann wird dazu die Lösung von 116,2 g (0,2 Mol) 2-Methyl-3-(2,4,6-trijod-3-aminobenzamido)-propionitril in 500 ml Dioxan gegeben.

Nach Rühren über Nacht wird das Dioxan im Vakuum abdestilliert, der Rückstand in etwa 1 l Wasser aufgenommen, über Kohle filtriert und das 2-Methyl-3-[2,4,6-trijod-3-(1-morpholinoäthylidenamino)-benzamido]-propionitril durch Zugabe von Ammoniak bei pH 8,5 ausgefällt. Das noch feuchte Produkt wird nach Filtration in 1,2 l 1 N HCl aufgenommen und über Kohle filtriert. Aus dem nunmehr klaren Filtrat wird das Nitril mit Lauge wieder gefällt und abfiltriert.

Man erhält 121 g (87,5% der Theorie) an 2-Methyl-3-[2,4,6-trijod-3-(1-morpholinoäthylidenamino)-benzamido]-propionitril vom Fp: 129-130°C.

*Beispiel 3*

250 ml Phosphoroxychlorid werden bei 0°C mit 19 g N-Acetylmorpholin versetzt. Dabei steigt die Temperatur trotz Kühlens auf 10°C an. Nach 5 Minuten werden unter weiterem Kühlen bei 5°C 29 g 2-Methyl-3-(2,4,6-trijod-3-aminobenzamido)-propionitril unter Rühren zugegeben. Es wird über Nacht bei 20°C weitergerührt, wobei eine fast klare Lösung entsteht.

Zur Aufarbeitung wird das Phosphoroxychlorid so gut wie möglich im Vakuum abdestilliert und der Eindampfrest in 150 ml Chloroform gelöst und in 300 ml Wasser eingegossen. Dann wird wie in Beispiel 1 beschrieben weiter aufgearbeitet.

Man erhält 33 g (95,4% der Theorie) 2-Methyl-3-[2,4,6-trijod-3-(1-morpholinoäthylidenamino)-benzamido]-propionitril vom Fp: 129-130°C.

*Beispiel 4*

46 g Phosphoroxychlorid in 200 ml Toluol werden bei 0°C unter Kühlen mit 19,4 g N-Acetylmorpholin versetzt. Nach kurzer Zeit bildet sich ein weisser Niederschlag in der Lösung. Unter Rühren versetzt man dann mit 29 g 2-Methyl-3-(2,4,6-trijod-3-aminobenzamido)-propionitril und lässt 40 Stunden bei 20°C weiterrühren. Das dabei entstehende ölige Reaktionsprodukt wird in 400 ml Wasser aufgenommen und die leicht trübe Lösung über Kohle filtriert. Aus dem klaren sauren Filtrat wird durch Einstellen eines pH-Wertes von —8 das 2-Methyl-3-[2,4,6-trijod-3-(1-morpholinoäthylidenamino)-benzamido]-propionitril gefällt.

Man erhält 33,2 g, das sind 96% der Theorie. Schmelzpunkt: 129-130°C.

*Beispiel 5*

183,6 g(1,2 Mol) Phosphoroxychlorid werden, wie in Beispiel 1 beschrieben, bei einer Temperatur von 0°C mit 77,4 g (0,6 Mol) N-Acetylmorpholin und 116,2 g (0,2 Mol) 2-Methyl-3-(2,4,6-trijod-3-aminobenzamido)-propionitril gemischt und dann 18 Stunden unter Rückfluss gekocht. Die Aufarbeitung erfolgt wie in Beispiel 1 beschrieben.

Man erhält 121,3 g 2-Methyl-3-[2,4,6-trijod-3-(1-morpholinoäthylidenamino)-benzamido]-propionitril, das sind 87,6% der Theorie.

Fp: 129-130°C.

*Beispiel 6*

34 g 2,4,6-Trijod-3-(1-morpholinoäthylidenamino)-benzoylchloridhydrochlorid (hergestellt nach der Vorschrift gemäss DE-PS 2 235 935) werden in Chloroform suspendiert. Durch Zugabe von Triäthylamin wird die Base freigesetzt, worauf 5 g 3-Amino-isobuttersäurenitril zugegeben werden und das Gemisch 3 Stunden unter Rückfluss gekocht wird. Die so erhaltene Chloroformlösung wird dann im Vakuum eingedampft, der ölige Rückstand wird in 4 N Salzsäure aufgenommen, mit Aktivkohle behandelt und diese abfiltriert. Das saure Filtrat wird dann durch Zugabe von 40%iger NaOH auf pH 6 gebracht, dann wird mit konzentriertem wässrigem Ammoniak ein pH-Wert von 8,5 eingestellt. Der dabei ausgeschiedene Niederschlag wird einige Zeit bei 60°C stehen gelassen, dann abgesaugt und getrocknet.

Man erhält 34,5 g an 2-Methyl-3-[2,4,6-trijod-3-(1-morpholinoäthylidenamino)-benzamido]-propionitril, das sind 93,6% der Theorie.

*Beispiel 7*

137 g des nach den vorhergehenden Beispielen erhaltenen 2-Methyl-3-[2,4,6-trijod-3-(1-morpholinoäthylidenamino)-benzamido]-propionitril werden in 300 ml konzentrierter Salzsäure gelöst und auf 80°C erhitzt. Nach 2 Stunden ist die Hydrolyse beendet und die wässrig-salzsaure Lösung wird im Vakuum eingedampft. Der Rückstand wird in wässriger Natronlauge gelöst und von unlöslichen Anteilen abfiltriert. Aus dem Filtrat wird die Säure durch Einstellen eines pH-Wertes von 4,5 gefällt.

Man erhält 123 g (87,6% der Theorie) an 2-Methyl-3-[2,4,6-trijod-3-(1-morpholinoäthylidenamino)-benzamido]-propionsäure. Durch Einengen der Mutterlauge werden noch weitere 16,2 g Säure (11,5%) erhalten. Nach Umkristallisation der vereinigten Produkte aus Methanol erhält man 124 g reine kristalline Säure vom Fp: 202-205°C, das sind 87,2% der Theorie bezogen auf 2-Methyl-3-(2,4,6-trijod-3-aminobenzamido)-propionitril, wenn die Herstellungsweise gemäss Beispiel 1 für die Herstellung des Nitrils diente.

*Beispiel 8*

34,5 g 2-Methyl-3-[2,4,6-trijod-3-(1-morpholinoäthylidenamino)-benzamido]-propionitril werden in 1 l 96%igem Alkohol heiss gelöst und nach Zugabe von 20 g 20%iger Natronlauge 2 Stunden unter Rückfluss gekocht. Danach wird der Alkohol abgezogen, der Rückstand mit Wasser auf 500 ml verdünnt und mit Salzsäure auf pH 1 eingestellt. Dabei sich abscheidende Flocken werden abfiltriert. Aus dem klaren Filtrat wird die Säure durch Einstellen des pH auf 4,5 ausgefällt und abfiltriert. Nach Umkristallisieren aus Methanol erhält man 25 g reine kristalline 2-Methyl-3-[2,4,6-trijod-3-(1-morpholinoäthylidenamino)-benzamido]-propionsäure vom Fp: 202-205°C, das sind 72% der Theorie, bezogen auf 2-Methyl-3-(2,4,6-trijod-3-aminobenzamido)-propionitril, wenn die Herstellung des Nitrils gemäss Beispiel 1 erfolgte.

*Beispiel 9*

30 g 2-Methyl-3-[2,4,6-trijod-3-(1-morpholinoäthylidenamino)-benzamido]-propionitril werden in 150 ml 10 N methanolischer Salzsäure 2 Stunden unter Rückfluss gekocht. Die methanolische Salzsäure wird vollständig abdestilliert und der Eindampfrest in 300 ml Wasser aufgenommen, kurz aufgekocht und von Unlöslichem abfiltriert. Durch Einstellen eines pH-Wertes von 4,5 wird die Säure ausgefällt und isoliert. Man erhält nach Umkristallisieren aus Methanol 24,2 g 2-Methyl-3-[2,4,6-trijod-3-(1-morpholinoäthylidenamino)-benzamido]-propionsäure vom Fp: 202-205°C. Die Ausbeute beträgt, bezogen auf 2-Methyl-3-(2,4,6-trijod-3-aminobenzamido)-propionitril und unter Zugrundelegung der Herstellung des Nitrils nach Beispiel 1, 78% der Theorie.

**Patentansprüche für die Vertragsstaaten:**
BE, CH, DE, FR, GB, IT, LI, LU, NL, SE

1. 2-Methyl-3-[2,4,6-trijod-3-(1-morpholinoäthylidenamino)-benzamido]-propionitril.

2. Verfahren zur Herstellung der Verbindung gemäss Anspruch 1, dadurch gekennzeichnet, dass man in an sich bekannter Weise

a) 2-Methyl-3-(2,4,6-trijod-3-aminobenzamido)-propionitril mit N-Acetylmorpholin in Gegenwart von Phosphoroxychlorid umsetzt und das erhaltene säurehaltige Reaktionsprodukt in Wasser aufnimmt, oder

b) 2,4,6-Trijod-3-(1-morpholinoäthylidenamino)-benzoylchlorid mit 3-Aminoisobuttersäurenitril in einem inerten organischen Lösungsmittel umsetzt und das erhaltene Reaktionsprodukt mit wässriger Säure behandelt,

worauf die freie Base durch Alkalisieren auf einen pH-Wert von mindestens 8 ausgefällt wird.

3. Verwendung der Verbindung gemäss Anspruch 1 als Zwischenprodukt zur Herstellung von 2-Methyl-3-[2,4,6-trijod-3-(1-morpholinoäthylidenamino)-benzamido]-propionsäure, dadurch gekennzeichnet, dass sie der Hydrolyse durch saure oder alkalische Medien unterworfen wird.

**Patentansprüche für den Vertragsstaat:** AT

1. Verfahren zur Herstellung von 2-Methyl-3-[2,4,6-trijod-3-(1-morpholinoäthylidenamino)-

benzamido]-propionitril, dadurch gekennzeichnet, dass man in an sich bekannter Weise

a) 2-Methyl-3-(2,4,6-trijod-3-aminobenzamido)-propionitril mit N-Acetylmorpholin in Gegenwart von Phosphoroxychlorid umsetzt und das erhaltene säurehaltige Reaktionsprodukt in Wasser aufnimmt, oder

b) 2,4,6-Trijod-3-(1-morpholinoäthylidenamino)-benzoylchlorid mit 3-Aminoisobuttersäurenitril in einem inerten organischen Lösungsmittel umsetzt und das erhaltene Reaktionsprodukt mit wässriger Säure behandelt,

worauf die freie Base durch Alkalisieren auf einen pH-Wert von mindestens 8 ausgefällt wird.

2. Verwendung von gemäss Anspruch 1 erhaltenem 2-Methyl-3-[2,4,6-trijod-3-(1-morpholinoäthylidenamino)-benzamido]-propionitril als Zwischenprodukt zur Herstellung von 2-Methyl-3-[2,4,6-trijod-3-(1-morpholinoäthylidenamino)-benzamido]-propionsäure, dadurch gekennzeichnet, dass es der Hydrolyse durch saure oder alkalische Medien unterworfen wird.

**Revendications pour les Etats Contractants:** BE, CH, DE, FR, GB, IT, LI, LU, NL, SE

1. 2-Méthyl-3-[2,4,6-triiodo-3-(1-morpholinoéthylidèneamino)-benzamido]-propionitrile.

2. Procédé pour la production du composé suivant la revendication 1, caractérisé en ce que, de manière en soi connue:

a) on fait réagir du 2-méthyl-3-(2,4,6-triiodo-3-aminobenzamido)-propionitrile avec de la N-acétylmorpholine en présence d'oxychlorure de phosphore et on reprend le produit de réaction contenant un acide obtenu dans l'eau, ou bien

b) on fait réagir du chlorure de 2,4,6-triiodo-3-(1-morpholinoéthylidèneamino)-benzoyle avec du nitrile d'acide 3-aminoisobutyrique dans un solvant organique inerte et on traite le produit de réaction obtenu avec de l'acide aqueux,

après quoi on précipite la base libre par alcalinisation à un pH d'au moins 8.

3. Application du composé suivant la revendication 1 à titre d'intermédiaire pour la préparation d'acide 2-méthyl-3-[2,4,6-triiodo-3-(1-morpholinoéthylidèneamino)-benzamido]-propionique, caractérisé en ce qu'on le soumet à une hydrolyse avec des agents acides ou alcalins.

**Revendications pour l'Etat Contractant:** AT

1. Procédé pour la production de 2-méthyl-3-[2,4,6-triiodo-3-(1-morpholinoéthylidèneamino)-benzamido]-propionitrile, caractérisé en ce que, de manière en soi connue:

a) on fait réagir du 2-méthyl-3-(2,4,6-triiodo-3-aminobenzamido)-propionitrile avec de la N-acétylmorpholine en présence d'oxychlorure de phosphore et on reprend le produit de réaction contenant un acide obtenu dans l'eau, ou bien

b) on fait réagir du chlorure de 2,4,6-triiodo-3-(1-morpholinoéthylidèneamino)-benzoyle avec du nitrile d'acide 3-aminoisobutyrique dans un solvant organique inerte et on traite le produit de la réaction obtenu avec de l'acide aqueux,

après quoi on précipite la base libre par alcalinisation à un pH d'au moins 8.

2. Application du 2-méthyl-3-[2,4,6-triiodo-3-(1-morpholinoéthylidèneamino)-benzamido]-propionitrile obtenu suivant la revendication 1 comme intermédiaire pour la préparation d'acide 2-méthyl-3-[2,4,6-triiodo-3-(1-morpholinoéthylidèneamino)-benzamido]-propionique, caractérisé en ce qu'on le soumet à une hydrolyse avec des agents acides ou alcalins.

**Claims for the Contracting States:** BE, CH, DE, FR, GB, IT, LI, LU, NL, SE

1. 2-Methyl-3-[2,4,6-triiodo-3-(1-morpholinoethylidenamino)-benzamido]-propionitrile.

2. Process for the preparation of the compound according to claim 1, characterised in that, in a manner which is known per se,

a) 2-methyl-3-(2,4,6-triiodo-3-aminobenzamido)-propionitrile is reacted with N-acetylmorpholine in the presence of phosphorus oxychloride and the resulting acid-containing reaction product is taken up in water, or

b) 2,4,6-triiodo-3-(1-morpholinoethylidenamino)-benzoyl chloride is reacted with 3-aminoisobutyronitrile in an inert organic solvent and the resulting reaction product is treated with aqueous acid,

after which the free base is precipitated by rendering the mixture alkaline to a pH value of at least 8.

3. Use of the compound according to claim 1 as an intermediate product for the preparation of 2-methyl-3-[2,4,6-triiodo-3-(1-morpholinoethylideneamino)-benzamido]-propionic acid, characterised in that it is subjected to hydrolysis by acid or alkaline media.

**Claims for the Contracting State:** AT

1. Process for the preparation of 2-methyl-3-[2,4,6-triiodo-3-(1-morpholinoethylideneamino)-benzamido]-propionitrile, characterised in that, in a manner which is known per se,

a) 2-methyl-3-(2,4,6-triiodo-3-aminobenzamido)-propionitrile is reacted with N-acetylmorpholine in the presence of phosphorus oxychloride and the resulting acid-containing reaction product is taken up in water, or

b) 2,4,6-triiodo-3-(1-morpholinoethylideneamino)-benzoyl chloride is reacted with 3-aminoisobutyronitrile in an inert organic solvent and the resulting reaction product is treated with aqueous acid,

after which the free base is precipitated by rendering the mixture alkaline to a pH value of at least 8.

2. Use of 2-methyl-3-[2,4,6-triiodo-3-(1-morpholinoethylideneamino)-benzamido]-propionitrile obtained according to claim 1 as an intermediate product for the preparation of 2-methyl-3-[2,4,6-triiodo-3-(1-morpholinoethylideneamino)-benzamido]-propionic acid, characterised in that it is subjected to hydrolysis by acid or alkaline media.